(19) Europäisches Patentamt
European Patent Office
Office européen des brevets

(11) EP 3 954 826 B1

(12) **EUROPEAN PATENT SPECIFICATION**

(45) Date of publication and mention of the grant of the patent:
**17.01.2024 Bulletin 2024/03**

(21) Application number: **20787886.9**

(22) Date of filing: **07.04.2020**

(51) International Patent Classification (IPC):
***D21H 21/36*** (2006.01) ***A61F 13/511*** (2006.01) ***A61F 13/84*** (2006.01)

(52) Cooperative Patent Classification (CPC):
**D21H 21/36; A61F 13/511; A61F 13/51113; A61F 13/84;** A61F 2013/51143; A61F 2013/8414

(86) International application number:
**PCT/JP2020/015605**

(87) International publication number:
**WO 2020/209238 (15.10.2020 Gazette 2020/42)**

(54) **ANTIBACTERIAL PAPER AND METHOD FOR MANUFACTURING SAME**

ANTIBAKTERIELLES PAPIER UND VERFAHREN ZU SEINER HERSTELLUNG

PAPIER ANTIBACTÉRIEN ET SON PROCÉDÉ DE FABRICATION

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **08.04.2019 JP 2019073764**
**21.02.2020 JP 2020027895**

(43) Date of publication of application:
**16.02.2022 Bulletin 2022/07**

(73) Proprietor: **Kao Corporation**
**Chuo-ku**
**Tokyo 103-8210 (JP)**

(72) Inventors:
- **ITOI, Takashi**
**Haga-gun, Tochigi 321-3497 (JP)**
- **SUZUKI, Yuka**
**Haga-gun, Tochigi 321-3497 (JP)**

(74) Representative: **Vossius & Partner Patentanwälte Rechtsanwälte mbB**
**Siebertstraße 3**
**81675 München (DE)**

(56) References cited:
**EP-A1- 1 865 106**
**WO-A1-2016/143217**
**CN-A- 107 119 502**
**CN-A- 107 905 040**
**CN-A- 108 978 347**
**GB-A- 2 561 421**
**JP-A- H0 931 366**
**JP-A- H08 164 191**
**JP-A- 2015 089 487**
**JP-A- 2016 169 446**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

EP 3 954 826 B1

## Description

[Technical Field]

The present invention relates to an antibacterial paper containing an antibacterial agent.

[Background Art]

Conventionally, an antibacterial agent has been added to absorbent products such as disposable diapers and sanitary napkins in order to reduce unpleasant odor derived from the excrement during wearing and the like. Patent Literature 1 discloses a component of absorbent products, for example, a paper having antibacterial performance usable as a core-wrap sheet covering an absorbent core. In an example in Patent Literature 1, an application liquid containing an antibacterial agent is manufactured using water having a hardness in a predetermined range and the application liquid is applied to a wet paper web to manufacture a paper having antibacterial performance.

[Citation List]

[Patent Literature]

[Patent Literature 1] JP 2016-168133 A

[Summary of Invention]

The present invention relates to an antibacterial paper containing an antibacterial agent and calcium.

Preferably, according to the antibacterial paper of the present invention, the calcium content per 0.1 g of the antibacterial paper is 500 ppm or more and 800 ppm or less.

The present invention also relates to a method for manufacturing an antibacterial paper, having a papermaking step of forming a wet paper web by wet papermaking using a papermaking water having a Water hardness of 5° dH or more.

Preferably, the manufacturing method of the present invention has an antibacterial agent applying step of applying an antibacterial agent-containing liquid prepared from an antibacterial agent and a dilution water thereof to the wet paper web.

Preferably, the manufacturing method of the present invention has a drying step of heating and drying the wet paper web after undergoing the antibacterial agent applying step.

Preferably, in the manufacturing method of the present invention, the Water hardness of the dilution water is equal to or less than the Water hardness of the papermaking water.

The other characteristics of the present invention will be clear from the claims and the following description.

[Detailed Description of the Invention]

During wearing of an absorbent product, an absorbent core is deformed by external force such as body pressure of a wearer and swells with absorption of a body fluid such as excreted urine, so that a core-wrap sheet covering the absorbent core is required to be flexible and pliable in order to follow the deformation and swelling of the absorbent core. Also, components of the absorbent product other than the core-wrap sheet are required to have flexibility in many cases.

The present invention relates to provision of a flexible antibacterial paper having antibacterial performance. The present invention also relates to a method for manufacturing an antibacterial paper, which enables a flexible antibacterial paper having antibacterial performance to be efficiently manufactured.

In general, water used in wet papermaking in a papermaking plant (hereinafter also referred to as "papermaking water") usually has a Water hardness of 2 to 4.5° dH. Also, in general, use of water having a high hardness tends to be avoided due to concerns on clogging in water piping resulting from scale formation. As a result of various studies, the present inventor has found that a flexible paper can be obtained by wet papermaking according to a usual method using a papermaking water having a higher Water hardness than a usual papermaking water. The present inventor has also found that the flexible paper manufactured using the papermaking water having a high hardness has a higher calcium content in comparison with a paper manufactured using a normal papermaking water.

In general, it is known that coexistence of an antibacterial agent and a metal ion causes the metal ion to impair the antibacterial performance of the antibacterial agent. For example, it is known that the effect (sterilization performance) of an invert soap such as a benzalkonium salt represented by a formula (1) described below tends to be affected by the hardness of water. According to W.S. Mueller and O. B. Seely [Soap & Sanitary Chemicals, 27, No.6, 131(1951)], divalent and trivalent metal ions have greater impeding power to the sterilization performance of an invert soap than a monovalent

metal ion, and the ratios of the impeding power are as follows: (monovalent metal ion):(divalent metal ion):(trivalent metal ion)=1:100:1000. Also, in a general case of manufacturing a paper having antibacterial performance, an antibacterial agent is typically diluted with water which is the same as the papermaking water and used. If a high hardness water having a Water hardness of 5° dH or more as papermaking water, i.e., dilution water is used, the high hardness water contains a relatively large amount of metal ions (calcium ions, magnesium ions, etc.). As a result, opportunities of generation of metal salts (scum) through a reaction of the antibacterial agent and the metal ions derived from the dilution water increase, so that the antibacterial performance as designed cannot be obtained. As a result of various studies of the problem, the present inventor has found that both of the antibacterial activity and the flexibility of a paper can be obtained by separating the papermaking water and the dilution water, with the Water hardness of the latter set equal to or less than that of the former.

The antibacterial paper of the present invention contains an antibacterial agent. The antibacterial performance of the antibacterial paper of the present invention is exhibited by the antibacterial agent which the antibacterial paper contains. As the antibacterial agent used in the present invention, ones which can suppress the proliferation of bacteria can be used without particular limitation, and ones which have an effect for extinguishing the source of odor, i.e., ones which have effect for suppressing the proliferation of normal bacteria of skin, intestinal bacteria, and enzymes derived therefrom causing odor, are preferred. For example, an antibacterial agent which suppresses or extinguishes the proliferation and growth of the bacteria involved in the generation of urine odor can be used. Examples of the antibacterial agents used in the present invention include one or more selected from inorganic antibacterial agents, organic antibacterial agents, and the like.

Examples of the inorganic antibacterial agents include one or more selected from fine powder and needle crystals including antibacterial metal ions and salts of, for example, silver, zinc, copper, iron, magnesium, calcium, aluminum, antimony and bismuth supported on a carrier.

Examples of the carriers include one or more selected from zeolite, silica gel, low molecular weight glass, calcium phosphate, zirconium phosphate, silicates and titanium oxide.

Examples of the organic antibacterial agents include one or more selected from a cationic antibacterial agent, an anionic antibacterial agent, and a nonionic antibacterial agent.

Examples of the anionic antibacterial agents include one or more selected from piroctone olamine[1-hydroxy-4-methyl-6-(2,4,4-trimethylpentyl)-2(1H)-pyridone monoethanol amine salt] potassium oleate, sodium 1-pentasulfonate, sodium 1-decanesulfonate, sodium butylnaphthalenesulfonate, sodium dodecylsulfate, sodium dodecylbenzenesulfonate, and sodium hexadecylsulfate.

Examples of the nonionic antibacterial agents include one or more selected from chlorhexidine hydrochloride, chlorhexidine gluconate, triclocarban, triclosan, isopropylmethylphenol, halocarbon, and paraoxybenzoate ester.

It is preferable that the antibacterial agent used in the present invention contain an organic antibacterial agent. The reason is that an organic antibacterial agent has higher antibacterial effect in comparison with an inorganic antibacterial agent such as zinc oxide and a silver-containing antibacterial agent. Further, an inorganic antibacterial agent is typically insoluble in water, so that when an antibacterial agent is applied to a paper by a non-contact applying method such as spraying with a spray, use of an organic antibacterial agent as antibacterial agent is preferred from the viewpoint of stable supply.

As described below, in manufacturing of the antibacterial paper of the present invention, an antibacterial agent-containing liquid prepared by diluting an antibacterial agent with a dilution water is used. It is therefore preferable that the antibacterial agent used in the present invention be soluble in water to an extent or dispersible in water, from the viewpoint of efficient manufacturing of the antibacterial paper with easy preparation of the antibacterial agent-containing liquid, the viewpoint of prevention of clogging in the step of spraying the antibacterial agent-containing liquid and the like. Specifically, the antibacterial agent used in the present invention has a residual ratio measured by the following method of preferably less than 0.5 mass%, more preferably 0.3 mass% or less, and even more preferably 0 mass%. In other words, the ratio is 0 mass% or more. Note that in manufacturing examples described below, two types of antibacterial agents A and B were used, and the antibacterial agent A had a residual ratio of 0.01 mass%, and the antibacterial agent B had a residual ratio of 0.005 mass%.

<Method for measuring residual ratio>

Into a beaker having a capacity of 100 mL, 10 g of an antibacterial agent to be measured and 90 g of deionized water (water temperature: 20°C) are placed and sufficiently mixed to prepare an antibacterial agent-containing liquid. The antibacterial agent-containing liquid is left to stand still in an environment at an atmospheric temperature of 25°C for 1 hour, and then poured onto a wire screen having 180-mesh openings. The mass of residues not passing through the wire screen is measured, and the measured value is defined as an amount of residue M1. From the amount of residue M1 and an initial mass of the antibacterial agent M0 (=10 g), the residual ratio of the antibacterial agent (mass%) is calculated based on the following formula:

$$\text{Residual ratio (mass\%)} = (M1/M0) \times 100$$

Preferred examples of the antibacterial agent for use in the present invention include a cationic antibacterial agent, which is one of organic antibacterial agents. A cationic antibacterial agent as the antibacterial agent contained in an antibacterial paper effectively suppresses the proliferation of bacteria derived from excrement, so that the inconvenience caused by the proliferation of the bacteria can be effectively suppressed.

The effect is prominent when the antibacterial paper of the present invention is used as an antibacterial paper for absorbent products, i.e., as a component of absorbent products such as disposable diapers (e.g., core-wrap sheet). For example, generation of the unpleasant odor during wearing and skin troubles of a wearer can be effectively suppressed.

The cationic antibacterial agent has an effect for suppressing the proliferation of microorganisms and enzymes derived from the microorganisms as the generation source of unpleasant odor components in the liquid phase of an excrement such as urine. Due to the effect of the cationic antibacterial agent, the generation source of odor components in an absorbent product is extinguished, so that deodorization effect can be exhibited.

Such a biological deodorant effect of the cationic antibacterial agent is effective against a wide variety of odor components ranging from acid to alkali, such as lower fatty acids, phenols, mercaptans, ketones, aldehydes, and amines.

Among the cationic antibacterial agents, ones containing a quaternary ammonium salt is preferred, because high antibacterial activity can be exhibited even with a low concentration.

The quaternary ammonium salt can be used without any particular limitation, and examples thereof include an alkylpyridinium salt, a benzethonium salt, a benzalkonium salt, a monoalkyltrimethyl ammonium salt, and a dialkyldimethyl ammonium salt, from which one or more may be selected for use.

Among the cationic antibacterial agents containing a quaternary ammonium salt, ones containing one or more quaternary ammonium salts selected from cetylpyridinium chloride, benzethonium chloride, dequalinium chloride, didecyldimethylammonium chloride, benzalkonium cetyl phosphate, and benzalkonium chloride are particularly preferred.

Among benzalkonium salts, a benzalkonium salt represented by the following formula (1) is particularly preferred, because high antibacterial activity can be exhibited with a further lower concentration.

[Chem. 1]

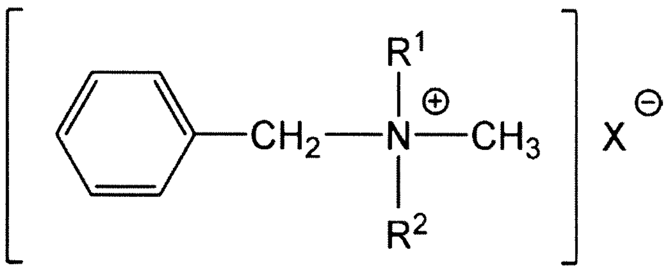

(1)

In the formula (1), $R^1$ and $R^2$ are the same or different and each represent a methyl group, an ethyl group, a straight-chain or branched-chain alkyl group having 8 or more and 20 or less carbon atoms, or a straight-chain or branched-chain alkenyl group having 8 or more and 20 or less carbon atoms; and $X^-$ represents a monovalent anion.

Ones represented by the formula (1) may be used alone or in combination of two or more.

Examples of the preferred combination of $R^1$ and $R^2$ in the formula (1) include a combination of $R^1$ being a methyl group and $R^2$ being a straight-chain or branched-chain alkyl group having 8 or more and 20 or less, and preferably 8 or more and 18 or less carbon atoms.

In addition, examples thereof include a combination of $R^1$ and $R^2$ which are the same group, and the group is a straight-chain or branched-chain alkyl group having 8 or more and 20 or less, and preferably 8 or more and 18 or less carbon atoms.

In the formula (1), it is preferable that the monovalent anion represented by $X^-$ be, for example, a halide ion or an anionic active group. The "anionic active group" refers to an ion having an anionic surface active properties.

It is preferable that the anionic active group have 6 or more carbon atoms, and preferably 10 or more carbon atoms.

It is also preferable that the anionic active group have 20 or less, and preferably 18 or less carbon atoms.

It is preferable that the anionic active group for use be an anionic active group containing a straight-chain or branched-chain alkyl group or a straight-chain or branched-chain alkenyl group.

It is preferable that the anionic active group for use be one containing one or more selected from, for example, an alkyl phosphate ester salt, an alkyl carboxylate salt, an alkyl sulfonate salt, and an alkyl sulfate salt, from the viewpoint of antibacterial ability.

It is particularly preferable to use one containing an alkyl phosphate represented by the following formula (2) from the viewpoint of safety (low irritation to the skin).

[Chem. 2]

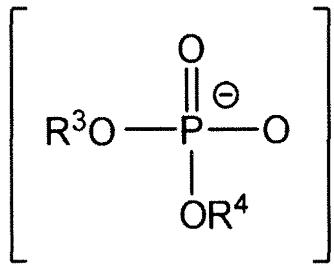

(2)

In the formula (2), one of $R^3$ and $R^4$ represents a straight-chain or branched-chain alkyl group having 6 or more and 20 or less carbon atoms or a straight-chain or branched-chain alkenyl group having 6 or more and 20 or less carbon atoms, and another represents a hydrogen atom, a methyl group or an ethyl group.

Examples of the preferred combination of $R^3$ and $R^4$ include a combination of $R^3$ being a hydrogen atom and $R^4$ being a straight-chain or branched-chain alkyl group having 8 or more and 20 or less, and preferably 8 or more and 18 or less carbon atoms. Having a cationic active group (quaternary amine) of a long chain alkyl, the texture (bulkiness and flexibility) of the antibacterial paper can be improved.

Among the benzalkonium salts described above, it is particularly preferable to use one or more selected from benzalkonium chloride and benzalkonium cetyl phosphate from the viewpoints of high antibacterial activity, safety and fast-acting property.

Among organic cationic antibacterial agents, benzalkonium cetyl phosphate is particularly preferred due to relatively high safety with good balance between the antibacterial activity and the low irritation to the skin (low solubility in water).

Due to this, the use of the antibacterial paper of the present invention as an antibacterial paper for absorbent products, i.e., a component (e.g. core-wrap sheet) of the absorbent product, is particularly effective.

As benzalkonium chloride, an antibacterial agent sold with a trade name "Sanisol B-50" from Kao Corporation may be used, and as benzalkonium cetyl phosphate, an antibacterial agent sold with a trade name "Sanisol P-2" from Kao Corporation may be used.

The antibacterial paper of the present invention has an antibacterial activity value of preferably 1 or more, more preferably 1.5 or more, and even more preferably 2 or more, the antibacterial activity value being measured according to the bacteria solution absorption method in JIS L 1902: 2015 "Determination of antibacterial activity and efficacy of textile products".

Also, the antibacterial activity value is practically 6 or less.

The antibacterial activity value is an index of the antibacterial performance of an antibacterial paper, and the antibacterial performance is evaluated higher as the value increases. An antibacterial paper having an antibacterial activity value equal to or larger than the lower limit described above can effectively suppress the proliferation of bacteria.

In particular, since the proliferation of bacteria derived from an excrement such as urine can be effectively suppressed, the antibacterial paper is particularly suitable as an antibacterial paper for absorbent products, i.e., a component (e.g. core-wrap sheet) of absorbent products.

A specific method for measuring the antibacterial activity value is as follows.

<Method for measuring antibacterial activity value>

The measurement is performed according to the bacteria solution absorption method in "Determination of antibacterial activity and efficacy of textile products" (quantitative test method including inoculating a test inoculation bacteria solution directly onto a test piece) described above, and in the case where no clear provisions are included in the JIS, the measurement is performed as follows.

6.5. Making of nutrient medium (NB)

To 1000 mL of a distilled water manufactured by Kanto Chemical Co., Inc., 8 g of a nutrient broth manufactured by Becton, Dickinson and Company is added and stirred well to obtain an aqueous solution. The aqueous solution is adjusted to a pH of 6.9$\pm$0.2, and then sterilized in an autoclave so as to obtain a nutrient medium (NB).

6.11. Making of agar medium (EA)

To 1000 mL of a distilled water manufactured by Kanto Chemical Co., Inc., 2.5 g of dehydrated yeast extract Bacto™ Yeast Extract manufactured by Becton, Dickinson and Company, 5.0 g of tryptone made from casein Bacto™ Tryptone manufactured by the same company, 1.0 g of D(+)glucose manufactured by FUJIFILM Wako Pure Chemical Corporation, and 12 to 15 g of agar manufactured by FUJIFILM Wako Pure Chemical Corporation are added, and stirred well to obtain an aqueous solution. The aqueous solution is adjusted to a pH of 7.2$\pm$0.2 and then sterilized in a high-pressure vapor sterilizer (CLG40L, autoclave) manufactured by ALP Co., Ltd. under conditions at a temperature of 120°C$\pm$2°C and a pressure of 103 kPa$\pm$5 kPa so as to obtain an agar medium (EA) for the pour plate culture method.

7. Test strain

Name of bacteria: colon bacillus, WDCM code: Escherichia coli NBRC 3301

7.2.4. Making of test strain (slant medium method)

The slant medium method is as follows. Into a 50-mL test tube manufactured by AS ONE Corporation, about 10 mL of the agar medium (EA) heated in advance to be melted was poured, and the open end of the test tube is blocked with a cotton plug manufactured by AS ONE Corporation. After completion of sterilization in an autoclave, the test tube is placed at an inclination angle of about 15 degrees to the horizontal plane in a clean room, so that the content is solidified. Ones with condensed water lost are dissolved and solidified again for use. The slant medium is thus made.

Next, the bacteria (colon bacillus) are transplanted to the slant medium so as to make a preculture A. The slant medium with the bacteria transplanted (preculture A) is subjected to cultivation at 37°C$\pm$2°C for 24 to 48 hours in a low-temperature thermostat IJ101W manufactured by Yamato Scientific Co., Ltd., and then stored at a temperature of 5 to 10°C. Note that in smearing with a platinum loop onto the slant medium, bacteria are dispersed in the condensed water shown in the test tube and a straight line is drawn therefrom toward above the slant. The tip end of the platinum loop is once released from the medium, and then immersed in the condensed water, again. A meandering line is drawn toward above the slant this time.

8.1.1.2. Preculture B

Into a conical flask with a lid having a capacity of 100 mL manufactured by AS ONE Corporation, 20 mL of the nutrient medium (NB) is placed. From a plate of the preculture A, a colony is scraped out with a platinum loop, and the colony is inoculated into the culture solution in the flask. A preculture B is thus made.

8.1.1.3. Preculture C

Into a conical flask with a lid having a capacity of 100 mL manufactured by AS ONE Corporation, 20 mL of the nutrient medium (NB) is placed. Into the conical flask with a lid, 0.4 mL of culture solution of the preculture B is added to make a preculture C.

8.1.2. Preparation of test inoculation bacteria solution

The bacteria concentration of the preculture C after cultivation is controlled to $1\times10^5$ to $3\times10^5$ CFU/mL using a nutrient medium (NB) 20-fold diluted with water at room temperature by a spectrophotometer (U-3310) manufactured by Hitachi High-Technologies Corporation. A test inoculation bacteria solution is thus prepared.

8.1.4. Testing operation

8.1.4.1. Inoculation onto test piece

As a test piece, an antibacterial paper to be measured is placed into a vial bottle, and the test inoculation

bacteria solution as an inoculation solution is inoculated to the test piece. Specifically, using Pipetman (P200) manufactured by Gilson, 0.2 mL per spot of the inoculation solution is inoculated onto a plurality of spots on a single side of the test piece in the vial bottle. In that case, contact of the inoculation solution with the wall surface and cap of the vial bottle is prohibited.

8.1.4.2. Washing out immediately after inoculation

Immediately after inoculation onto a test piece, 20 mL of normal saline solution is added into each vial bottle of 3 test sample specimens and 3 control sample specimens. The normal saline solution is prepared by adding 8.5 g of sodium chloride manufactured by FUJIFILM Wako Pure Chemical Corporation to 1000 mL of a distilled water manufactured by Kanto Chemical Co., Inc., stirring the mixture well, and then sterilizing the mixture in an autoclave.

Next, the cap of the vial bottle is tightened, and the washing out process (JIS L 1902: 2015: Appendix B) using a vortex mixer (TX-3000L manufactured by AS ONE Corporation) is performed. In the washing out process, the bottom of the vial bottle is pressed against a plate, a holder part made of rubber and 5-second shaking is performed 5 cycles.

Incidentally, in the case of measuring the antibacterial activity value of an antibacterial paper used as a component of absorbent products such as commercially available disposable diapers and various products, the following method can be employed. Using a dryer or a cold spray, the hot melt adhesive is made ineffective to carefully remove other components in the product, so that the antibacterial paper to be measured is obtained. The antibacterial activity value of the antibacterial paper thus obtained can be measured according to the bacteria solution absorption method in "Determination of antibacterial activity and efficacy of textile products." The means for taking the antibacterial paper out is commonly used in other measurements in the present specification.

The content of the antibacterial agent in the antibacterial paper of the present invention may be appropriately set corresponding to the use of the antibacterial paper and the like, such that a predetermined antibacterial performance can be exhibited. From the viewpoint of achieving the lower limit or more of the antibacterial activity value described above, the content of the antibacterial agent in the antibacterial paper of the present invention expressed as the mass per unit area (basis weight) is preferably 0.001 $g/m^2$ or more, and more preferably 0.01 $g/m^2$ or more. In general, as the content of the antibacterial agent increases, the antibacterial performance improves.

Also, from the viewpoints of the irritation to the skin and the permeability of excrement such as urine, the content of the antibacterial agent in the antibacterial paper of the present invention is preferably 0.2 $g/m^2$ or less, and more preferably 0.1 $g/m^2$ or less. In particular, in the case of using the antibacterial paper as a component of absorbent products, it is preferable that the content be equal to or less than the upper limit from the viewpoints of the irritation to the skin and the permeability of excrement such as urine.

According to the antibacterial paper of the present invention, the calcium content per 0.1 g of the antibacterial paper is 500 ppm or more. The present inventor has found that the calcium content of the paper can be an index of flexibility of the paper, and the paper has higher flexibility with increase in the calcium content.

With a calcium content per 0.1 g of the antibacterial paper of 500 ppm or more, for example, in the case of using the antibacterial paper as a core-wrap sheet to cover an absorbent core, when the absorbent core is deformed by body pressure or the like, or swells with absorption of an excrement such as urine, the antibacterial paper as core-wrap sheet can cause expansion or deformation to follow the deformation and swelling of the absorbent core, so that inconvenience such as fracture of the core-wrap sheet hardly occurs.

The calcium content per 0.1 g of the antibacterial paper of the present invention is preferably 510 ppm or more, and more preferably 515 ppm or more, from the viewpoint of improvement in the flexibility.

On the other hand, from the viewpoint of securing the paper strength with the hydrogen bonds between constituent fibers such as cellulose fibers of the antibacterial paper maintained, the upper limit of the calcium content per 0.1 g of the antibacterial paper of the present invention is 800 ppm or less, preferably 750 ppm or less, and more preferably 700 ppm or less.

It is feasible to control the calcium content per 0.1 g of the antibacterial paper to 500 ppm or more by using a water having a high hardness which is not usually used as papermaking water for wet papermaking in manufacturing of an antibacterial paper by the conventionally known wet papermaking method, and the details on this point are described later.

The calcium content of an antibacterial paper is measured by the following method.

<Method for measuring calcium content of paper>

A beaker made of Teflon (registered trademark) (manufactured by AS ONE Corporation) and a watch glass made of Teflon (registered trademark) are immersed in a nitric acid tank for 1 day or more. The beaker and the watch glass are then taken out from the nitric acid tank, washed with ultra-pure water, and then dried. Separately from the operation, the paper to be measured is cut into squares of 5 mm with a pair of ceramic scissors to obtain small pieces.

The pair of scissors is lightly washed with alcohol and water before use, and then the moisture thereon is wiped off. Using a balance, 0.1 g of the small pieces obtained are weighed out and recorded to the fourth decimal place. The small pieces weighed out are fractionated in the washed beaker and precisely weighed. Into the beaker, 30 mL of hydrochloric acid (manufactured by Kanto Chemical Co., Inc., for atomic absorption spectrometry) and 10 mL of nitric acid (manufactured by Kanto Chemical Co., Inc., for atomic absorption spectrometry) are placed. Then, the upper opening of the beaker is covered with the washed watch glass, and the beaker is placed on a hot plate. Under conditions with a hot plate temperature of 200 to 250°C, heat treatment is performed for 3 hours. During the heat treatment, the decomposition state of the content in the beaker is observed, such that nitric acid is appropriately added thereto. After the heat treatment, the watch glass is displaced to concentrate the content to 10 mL. After concentration, the beaker is put down from the hot plate so as to be cooled, and 2 mL of nitric acid is added to the content in the beaker. The inside of the watch glass is rinsed with a small amount of ultra-pure water (manufactured by FUJIFILM Wako Pure Chemical Corporation), and the ultra-pure water used in the rinsing is placed into the beaker. The content in the beaker is transferred into a 50-mL DigiTUBE washed with ultra-pure water. The inside of the beaker after transfer of the content is rinsed with a small amount of ultra-pure water, and the ultra-pure water used in the rinsing is transferred to a 50-mL DigiTUBE. The volume of the content (aqueous solution) in the 50-mL DigiTUBE is fixed at a volume of 50 mL, and the content is defined as a test solution. Calcium in the test solution is quantitively determined with an ICP-MS (Inductively Coupled Plasma Mass Spectrometer, manufactured by Agilent Technologies) apparatus.

The antibacterial paper of the present invention has a flexural rigidity value of preferably 30 cN or less, and more preferably 29 cN or less. With decrease in the flexural rigidity value, the antibacterial paper has more excellent flexibility, indicating pliable properties.

Also, the antibacterial paper of the present invention has a flexural rigidity value of practically 25 cN or more.

The method for measuring the flexural rigidity value is described later.

The antibacterial paper of the present invention is typically made of mainly fiber. The fiber content of the antibacterial paper of the present invention relative to the total mass of the antibacterial paper is at least 50 mass% or more, preferably 80 mass% or more, and more preferably 90 mass% or more.

Also, the fiber content of the antibacterial paper of the present invention is less than 100 mass%.

The antibacterial paper of the present invention contains at least an antibacterial agent other than fiber, and may further contain other components. Examples of the components other than fiber and the antibacterial agent which the antibacterial paper of the present invention may contain include various components used usually in wet papermaking such as a paper strength enhancer, a filler, a dye, a pigment, a pH adjuster, a yield improver, a water resistant additive, and a defoaming agent, and one of these may be used alone or two or more of them may be used in combination.

The antibacterial paper of the present invention is typically made of mainly cellulose fiber. As the cellulose fiber, ones usable as raw material of paper may be used without particular limitation. Examples thereof include natural fiber including wood pulp such as softwood pulp and hardwood pulp; non-wood pulp such as cotton pulp and hemp pulp; modified pulp such as cationized pulp and mercerized pulp; and regenerated fiber such as cupra and rayon; and one of these may be used alone or two or more of these may be mixed for use.

The freeness of the cellulose fiber used in the present invention is not particularly limited, preferably 500 mL or more, and more preferably 600 mL or more, from the viewpoint of balance between the strength and the liquid permeability of the antibacterial paper.

Also, the freeness of the cellulose fiber used in the present invention is preferably 700 mL or less, and more preferably 680 mL or less.

The freeness is a value indicated by Canadian standard freeness (C. S. F.) specified in JIS P8121, indicating the beating (mechanically beating and grinding fiber in the presence of water) degree of fiber. With decrease in the freeness value, the beating degree increases, indicating the progress of fibrillation with large damage to the fiber by beating. The beating of fiber may be performed for paper stock (slurry) with fiber dispersed according to a usual method using a known beating machine such as a beater and a disc refiner.

The antibacterial paper of the present invention may contain other fiber in addition to cellulose fiber. Examples of the other fibers include fusion bondable fiber. As the fusion bondable fiber, fiber which is melted and bond to each other by heating may be used. Specific examples of the fusion bondable fibers include a polyolefin fiber of polyethylene, polypropylene or polyvinyl alcohol, a polyester fiber, a polyethylene-polypropylene composite fiber, a polyethylene-polyester composite fiber, a low-melting point polyester-polyester composite fiber, a polyvinyl alcohol-polypropylene composite fiber having a hydrophilic fiber surface, and a polyvinyl alcohol-polyester composite fiber. In the case of using a composite fiber, any of a core-sheath type composite fiber and a side-by-side type composite fiber may be used. One of these fusion bondable fibers may be used alone, or two or more thereof may be used in combination.

The antibacterial paper of the present invention typically includes a substrate mainly made of fiber, and the substrate contains an antibacterial agent. The substrate may have a single-layer structure or a stacked structure with two or more layers stacked. Examples of another form of the antibacterial paper of the present invention include a form having a substrate and a coating layer stacked on a single surface or both surfaces of the substrate, in which the coating

layer may contain an antibacterial agent.

The basis weight of the antibacterial paper of the present invention is not particularly limited, and may be appropriately set according to use of the antibacterial paper and the like.

For example, from the viewpoint of the balance between the strength and the liquid permeability or the flexibility, the antibacterial paper (the substrate described above) has a basis weight of preferably 10 $g/m^2$ or more, and more preferably 12 $g/m^2$ or more.

From the same viewpoint, the basis weight is preferably 50 $g/m^2$ or less, and more preferably 35 $g/m^2$ or less.

In the case of using the antibacterial paper of the present invention as a component of absorbent products, or in the case of using as a core-wrap sheet covering the outer surface of an absorbent core, the range described above is preferred also from the viewpoint of the balance with various properties such as liquid permeability.

The antibacterial paper of the present invention is particularly useful as a component of absorbent products. The present invention includes absorbent products having the antibacterial paper of the present invention described above.

The absorbent product of the present invention typically includes a topsheet to form a surface facing the skin, a backsheet to form a surface not facing the skin, and a liquid-retentive absorbent member disposed between the topsheet and the backsheet.

The topsheet is typically liquid-permeable.

The backsheet is typically sparingly liquid permeable or water-repellent, or may be liquid-permeable.

The absorbent member typically includes an absorbent core and a core-wrap sheet covering the absorbent core.

The antibacterial paper of the present invention is preferably used as a core-wrap sheet, because the use is particularly effective.

Note that "the surface facing the skin" is a surface of an absorbent product or a component thereof (e.g., an absorbent core) facing the skin of a wearer wearing the absorbent product, i.e., the surface relatively adjacent to the skin of a wearer, and "the surface not facing the skin" is a surface of an absorbent product or a component thereof on the opposite side of the skin of a wearer wearing the absorbent product, i.e., the surface relatively remote from the skin of a wearer.

As the topsheet, the backsheet and the absorbent core, ones which are usually used in this type of absorbent products may be used without particular limitation, respectively.

The absorbent products of the present invention widely include products used in absorption of body fluid (urine, soft stool, menstrual blood, sweat, etc.) excreted from a human body, including, for example, a disposable diaper, a sanitary napkin, a sanitary shorts, and an incontinence pad.

In an embodiment of the absorbent products of the present invention, in the case of the core-wrap sheet made of the antibacterial paper of the present invention, it is preferable that at least the surface facing the skin and the surface not facing the skin of an absorbent core be covered with the core-wrap sheet (antibacterial paper of the present invention).

Examples of the other embodiments of the absorbent products of the present invention include a product further having an intermediate sheet (sublayer) lying between the topsheet and the absorbent member, the intermediate sheet being made of the antibacterial paper of the present invention.

The absorbent product of the present invention may be manufactured in the same manner as this type of absorbent products. Specific examples of the manufacturing method include combining various types of members such as a topsheet, a backsheet, an absorbent member (an absorbent core and a core-wrap sheet) and an intermediate sheet according to a usual method.

The manufacturing method of absorbent products of the present invention typically includes the step of combining the antibacterial paper of the present invention described above with one or a combination of two or more of other members other than the antibacterial paper (a manufacturing intermediate of the absorbent product).

Examples of the embodiment of the manufacturing method of absorbent products of the present invention include a step of stacking a core-wrap sheet with an absorbent core, a topsheet and a backsheet, when using the antibacterial paper of the present invention as a core-wrap sheet. The step of stacking the core-wrap sheet may be performed, for example, by covering the absorbent core with the core-wrap sheet to obtain an absorbent member and disposing the absorbent member between the topsheet and the backsheet.

Next, the method for manufacturing an antibacterial paper of the present invention (hereinafter also referred to simply as "manufacturing method") is described. The antibacterial paper of the present invention described above may be manufactured by the manufacturing method of the present invention. Note that the manufacturing method of the present invention is mainly described below on matters not mentioned in the description of the antibacterial paper of the present invention. To the manufacturing method of the present invention, the description of the antibacterial paper of the present invention is appropriately applied unless otherwise specified.

It is preferable that the manufacturing method of the present invention have a papermaking step of forming a wet paper web by wet papermaking and an antibacterial agent applying step of applying an antibacterial agent-containing liquid to the wet paper web. It is also preferable that the manufacturing method have a drying step of heating and drying

the wet paper web after undergoing the antibacterial agent applying step.

The manufacturing method of the present invention may be performed according to a usual method using a known wet papermaking machine. The wet papermaking machine typically includes a slurry preparation part to prepare a slurry, a forming part to continuously form a wet paper web from the slurry using a papermaking screen, a drying part to heat and dry the wet paper web, and a winder part to wind the heated and dried paper (antibacterial paper) into a roller shape. The papermaking step may be performed in the slurry preparation part and the forming part. The drying step may be performed in the drying part. The drying part of the wet papermaking machine includes heating and drying means such as a Yankee dryer.

The papermaking step typically includes a step of preparing a slurry containing fiber (aqueous dispersion of fiber) and a step of spreading the solid content in the slurry on a papermaking screen to form a wet paper web on the surface of the papermaking screen. In the slurry, a papermaking raw material usually used in wet papermaking such as a paper strength enhancer and a filler may be contained on an as needed basis. The solid content concentration of the slurry is usually about 0.005 to 0.2 mass%. The papermaking step may be performed according to a usual method using the conventionally known wet papermaking machine.

In the papermaking step described above, a wet paper web is formed using a papermaking water having a Water hardness of 5° dH or more. In other words, for example, in the case where the manufacturing method of the present invention is performed using a typical wet papermaking machine, as the water used in at least both part of the slurry preparation part and the forming part (papermaking water), a water having a Water hardness of 5° dH or more is used. As described above, in general, a Water hardness of a papermaking water used in a papermaking plant usually is 2 to 4.5° dH. Also, in general, use of a water having a high Water hardness tends to be avoided. In other words, in the manufacturing method of the present invention, a wet paper web is formed using a water having a higher hardness than a usual papermaking water. Formation of a wet paper web using a water having a Water hardness of 5° dH or more makes it easy to increase the calcium content of the antibacterial paper to be manufactured, so that the flexibility of the antibacterial paper can be increased as described above. The Water hardness of the papermaking water used in the papermaking step is preferably 5.5° dH or more, and more preferably 6° dH or more.

On the other hand, the upper limit of the Water hardness of the papermaking water used in the papermaking step is preferably 12° dH or less, more preferably 10° dH or less, and even more preferably 8° dH or less, from the viewpoint of securing the paper strength with the hydrogen bonds between constituent fibers such as cellulose fibers of the antibacterial paper maintained through control of the calcium content of the antibacterial paper in an appropriate range.

In the present invention, as the index of the hardness of water, Water hardness is used. In the present specification, Water hardness (° dH) refers to the hardness determined from the following formula based on the measurement of the concentration of calcium and magnesium in water in accordance with JIS K 0101: 1998 Testing method for industrial water, 15.1.2 Flame atomic absorption spectroscopy.

$$H\ (\text{mg CaO}/100\ \text{mL}) = \{(1.399 \times C_{Ca}) + (2.307 \times C_{Mg})\}/10$$

H: Water hardness
Cca: calcium concentration (mg CalL)
1.3999: factor for converting amount of calcium to calcium oxide equivalent (56.08/40.08)
$C_{Mg}$: magnesium concentration (mg Mg/L)
2.307: factor for converting amount of magnesium to calcium oxide equivalent (56.08/24.305)

In the antibacterial agent applying step, an antibacterial agent-containing liquid is applied to the wet paper web formed in the papermaking step.

The method of applying the antibacterial agent-containing liquid to the wet paper web is not particularly limited, and examples thereof include spraying with a spray (non-contact applying method) and coating with a known coating means (contact applying method).

The timing of applying the antibacterial agent-containing liquid may be immediately after formation of the wet paper web. In other words, the antibacterial agent-containing liquid may be applied immediately after spreading the slurry on the papermaking screen. Typically, the antibacterial agent-containing liquid is applied after squeeze dehydration of the wet paper web.

A typical wet papermaking machine includes a pressing means such as a press roller for squeeze dehydration of the wet paper web between a papermaking screen and a heating and drying means of a drying part. Immediately before applying the antibacterial agent-containing liquid, the wet paper web has a water content ratio of usually about 20 to 85 mass%.

The antibacterial agent-containing liquid is prepared by diluting an antibacterial agent (preferably a cationic antibacterial agent) with water. Although the antibacterial agent-containing liquid may contain components other than

the antibacterial agent and water on an as needed basis, the liquid typically contains the antibacterial agent and water only.

The content of the antibacterial agent in the antibacterial agent-containing liquid is not particularly limited, preferably 0.01 mass% or more, and more preferably 0.1 mass% or more, relative to the total mass of the antibacterial agent-containing liquid.

The content of the antibacterial agent in the antibacterial agent-containing liquid is preferably 5 mass% or less, and more preferably 2 mass% or less.

In the manufacturing method of the present invention, the Water hardness may be different between the water for preparing the antibacterial agent-containing liquid (dilution of antibacterial agent), i.e., "dilution water," and the water used in the papermaking step, i.e., "papermaking water." More specifically, in the manufacturing method of the present invention, the Water hardness of the dilution water is equal to or less than the Water hardness of the papermaking water, and preferably less than the Water hardness of the papermaking water.

The reason for the condition "Water hardness of dilution water ≤ Water hardness of papermaking water" and the reason for the preferred condition "Water hardness of dilution water < Water hardness of papermaking water" are as described above, i.e., to obtain an antibacterial paper achieving both of high antibacterial activity and high flexibility. Due to this, the antibacterial agent and the metal ions contained in the dilution water cause no reaction, so that an amount of the antibacterial agent required for exhibition of a specified antibacterial performance in the antibacterial paper to be manufactured can be surely applied. In a common technical knowledge in the art of wet papermaking, the same water is used in a series of steps (steps including preparing a slurry and forming and drying a wet paper web), and it is fairly uncommon to separately use water having a different hardness as in the present invention.

From the viewpoint of further surely exhibiting the effect described above, on the assumption that the Water hardness of the dilution water is equal to or less than the Water hardness of the papermaking water as described above, the Water hardness of the dilution water is preferably 4° dH or less, more preferably 3° dH or less, and even more preferably 0° dH, or 0° dH or more.

From the same viewpoint, regarding the ratio of the Water hardness of the dilution water to the Water hardness of the papermaking water on the assumption "Water hardness of dilution water ≤ Water hardness of papermaking water" as described above, the Water hardness of the dilution water/the Water hardness of the papermaking water is preferably 0.7 or less, more preferably 0.5 or less, and even more preferably 0, or 0 or more.

Examples

The present invention is further described in detail with reference to Examples, though the present invention is not limited thereto.

[Manufacturing Examples 1 to 14 and Comparative Manufacturing Examples 1 to 7]

Fiber (needle breached kraft pulp, NBKP) was dispersed in water to obtain a slurry, and the slurry was processed with a beating machine, so that the freeness of the NBKP was adjusted to 650 mL. Into the slurry, PAE (manufactured by Seiko PMC Corporation, trade name: "WS4030") as a wet paper strength enhancer was then fed at a concentration of 0.5 mass% relative to the dry mass of the total fiber in the slurry. The mixture was sufficiently stirred to make uniform distribution of each the components, so that a slurry with a solid content concentration of 0.2 mass% was prepared. Into a square sheet machine (250 mm square, manufactured by Kumagai Riki Kogyo Co., Ltd.), 10 L of a papermaking water was placed, and the slurry was then fed thereto for papermaking, so that a wet paper web was made. A filter paper (size: 28 cm by 28 cm) was placed on the wet paper web to adjust the water content ratio to 75 mass%. Next, an antibacterial agent was sprayed onto the wet paper web and then dried with a rotary dryer (manufactured by Kumagai Riki Kogyo Co., Ltd.), so that an antibacterial paper containing the antibacterial agent was manufactured.

In the wet papermaking, one type of water (papermaking water) having a specified Water hardness was used in the steps from slurry preparation to wet paper web formation. Also, an antibacterial agent-containing liquid using dilution water was sprayed onto the wet paper web with a spray, so that the antibacterial agent-containing liquid was applied to the wet paper web. Immediately before applying the antibacterial agent-containing liquid, the wet paper web had a water content ratio of 75 mass%. Also, in the wet papermaking, the papermaking conditions were appropriately set, such that the basis weight of the paper manufactured without spraying of the antibacterial agent-containing liquid was controlled to 16 g/m$^2$ in a dry state. The content of an antibacterial agent A was 0.01 g/m$^2$, and the content of an antibacterial agent B was 0.0075 g/m$^2$. As the antibacterial agent-containing liquid, the following antibacterial agent-containing liquid 1 or 2 was used.

(Papermaking water)

The Water hardness of the papermaking water used is shown in the following Table 1 and Table 2. The

papermaking water having a Water hardness of 1° dH or more was prepared by dissolving calcium chloride and magnesium chloride in deionized water to have a content mass ratio between calcium ions and magnesium ions (calcium ion:magnesium ion) of 7:3.

(Dilution water)

The Water hardness of the dilution water used is shown in the following Table 1 and Table 2. As the dilution water having a Water hardness of 0° dH, deionized water alone was used. The dilution water having a Water hardness of 1° dH or more was prepared by dissolving calcium chloride and magnesium chloride in deionized water to have a content mass ratio between calcium ions and magnesium ions (calcium ion:magnesium ion) of 7:3.

(Antibacterial agent-containing liquid 1)

· Composition: Antibacterial agent A/tromethamine/citric acid/water (dilution water)=3/0.7/0.3/96 was diluted by 10 times to have an antibacterial agent concentration of 0.3% in the antibacterial agent-containing liquid.
· Antibacterial agent A: cationic antibacterial agent (benzalkonium cetyl phosphate), manufactured by Kao Corporation, trade name: "Sanisol P-2"
· Tromethamine: manufactured by Angus Chemical Company, trade name: TRIS AMINOW Ultra PC Tromethamine
· Citric acid: manufactured by Fuso Chemical Co., Ltd., trade name: "Purified Citric Acid (anhydrous)"

(Antibacterial agent-containing liquid 2)

· Composition: Antibacterial agent B/water (dilution water)=3/97. In other words, the antibacterial agent concentration was 3% in the antibacterial agent-containing liquid.
· Antibacterial agent B: cationic antibacterial agent (benzalkonium chloride), manufactured by Kao Corporation, trade name: "Sanisol B-50"

A paper in Comparative Example 1 was obtained in the same manner as in the manufacturing step described above, except that the antibacterial agent-containing liquid was not sprayed.

The calcium content and the antibacterial activity value of the antibacterial papers obtained in Manufacturing Examples and Comparative Manufacturing Examples (Examples 1 to 17 and Comparative Examples 1 to 4) were measured by the methods described above, and the flexural rigidity value was also measured by the following method. The results are shown in Table 1 and Table 2.

<Method for measuring flexural rigidity value>

In the measurement, a handle-o-meter testing machine manufactured by Daiei Kagaku Seiki Mfg. Co., Ltd., complying with the rigidity measurement method specified in JIS L1096 (Testing methods for woven and knitted fabrics) was used. A test piece paper to be measured having a size of 250 mm by 250 mm was prepared. The slot width of the sample table of the testing machine was adjusted to 10 mm, and two of the test pieces were disposed horizontally with center portions thereof at the center position of the slot width, and the four corners of the test piece were fixed to the top face of the sample table with an adhesive tape. A blade was adjusted to descend from the top face of the sample table to a position below 7 mm therefrom (the lowest position). The blade was lowered at a constant rate of 200 mm/min from above the test piece, and the highest value (cN) indicated by the indicator (load cell) when the test piece was pressed with the blade was read. The measurement was performed 5 times, and the average was calculated to determine the flexural rigidity value of the paper. The measurement was performed at a room temperature of 23±2°C, and a relative humidity of 50% RH±5%.

[Table 1]

| | | Comparative Manufacturing Example | | | | | Manufacturing Example | | | | | | | | |
|---|---|---|---|---|---|---|---|---|---|---|---|---|---|---|---|
| | | 1 | 2 | 3 | 4 | 5 | 1 | 2 | 3 | 4 | 5 | 6 | 7 | 8 | 9 |
| Manufacturing method (conditions for wet papermaking) | Water hardness of papermaking water (°dH) | 4 | | | | | 5 | | | 6 | | | 8 | | |
| | Water hardness of dilution water (°dH) | | 0 | 4 | 8 | 12 | 0 | 4 | 5 | 0 | 4 | 5 | 0 | 4 | 8 |
| | Type of antibacterial agent | - | A | A | A | A | A | A | A | A | A | A | A | A | A |
| | Water hardness of dilution water/Water hardness of papermaking water | - | 0.0 | 1.0 | 2.0 | 3.0 | 0.0 | 0.8 | 1.0 | 0.0 | 0.7 | 0.8 | 0.0 | 0.5 | 1.0 |

(continued)

| | | Comparative Manufacturing Example | | | | | Manufacturing Example | | | | | | | | |
|---|---|---|---|---|---|---|---|---|---|---|---|---|---|---|---|
| | | 1 | 2 | 3 | 4 | 5 | 1 | 2 | 3 | 4 | 5 | 6 | 7 | 8 | 9 |
| Antibacterial paper | | Comparative Example 1 | Comparative Example 2 | Comparative Example 3 | Example 1 | Example 2 | Comparative Example 4 | Example 3 | Example 4 | Example 5 | Example 6 | Example 7 | Example 8 | Example 9 | Example 10 |
| | Content of antibacterial agent ($g/m^2$) | 0.01 | 0.01 | 0.01 | 0.01 | 0.01 | 0.01 | 0.01 | 0.01 | 0.01 | 0.01 | 0.01 | 0.01 | 0.01 | 0.01 |
| | Calcium content (ppm) | 420 | 420 | 470 | 505 | 570 | 465 | 515 | 528 | 510 | 560 | 585 | 600 | 650 | 670 |
| | Antibacterial activity value | | 5.7 | 1.5 | 1.2 | 0.5 | 5.5 | 1.5 | 1.3 | 4.2 | 1.4 | 12 | 3.5 | 1.6 | 1.2 |
| | Flexural rigidity value (cN) | 36.5 | 33.5 | 33.1 | 28.8 | 28.5 | 29.8 | 29.1 | 28.5 | 28.9 | 28.8 | 27.8 | 28.5 | 28.1 | 27.8 |

[Table 2]

| | | Comparative Manufacturing Example | Manufacturing Example | | | Comparative Manufacturing Example | Manufacturing Example | |
|---|---|---|---|---|---|---|---|---|
| | | 6 | 10 | 11 | 12 | 7 | 13 | 14 |
| Manufacturing method (conditions for wet papermaking) | Water hardness of papermaking water (° dH) | 8 | 12 | | | 4 | 5 | 12 |
| | Water hardness of dilution water (°dH) | 12 | 0 | 4 | 8 | 8 | 4 | 4 |
| | Type of antibacterial agent | A | A | A | A | B | B | B |
| | Water hardness of dilution water/Water hardness of papermaking water | 1.5 | 0.0 | 0.3 | 0.7 | 2.0 | 0.8 | 0.3 |
| Antibacterial paper | | Example 11 | Example 12 | Example 13 | Example 14 | Example 15 | Example 16 | Example 17 |
| | Content of antibacterial agent ($g/m^2$) | 0.01 | 0.01 | 0.01 | 0.01 | 0.0075 | 0.0075 | 0.0075 |
| | Calcium content (ppm) | 690 | 750 | 780 | 840 | 508 | 510 | 790 |
| | Antibacterial activity value | 0.5 | 2.5 | 1.2 | 1.0 | 1.5 | 4.5 | 3.5 |
| | Flexural rigidity value (cN) | 27.2 | 27.5 | 26.4 | 26.3 | 29.1 | 29.3 | 27.8 |

As described in Table 1 and Table 2, in Manufacturing Examples and Comparative Manufacturing Examples, 4 types of papermaking water each having a Water hardness of 4, 6, 8 and 12, were used. Using any of the papermaking water, with increase in the Water hardness of the dilution water, the calcium content of the antibacterial paper as resultant product increased, and accordingly, the flexural rigidity value of the antibacterial paper decreased, or the flexibility improved.

Compared with the antibacterial papers obtained in Comparative Manufacturing Examples 1 to 3 (antibacterial papers in Comparative Examples 1 to 3) and the antibacterial paper obtained in Manufacturing Example 1 (antibacterial paper in Comparative Example 4), the antibacterial papers in Examples stably had characteristics including antibacterial performance and flexibility.

Also, compared with the antibacterial papers obtained in Comparative Manufacturing Examples 1 to 5 using papermaking water having a Water hardness of 4° dH (antibacterial papers in Comparative Examples 1 to 3 and Examples 1 to 2) and in Comparative Manufacturing Example 6 using papermaking water having a Water hardness of 8° dH and dilution water having a Water hardness of 12° dH, flexible antibacterial papers having antibacterial performance were more stably manufactured in Manufacturing Examples. Focusing on the antibacterial activity value suggests that in the case of the Water hardness of the papermaking water is fixed, better antibacterial performance with a higher antibacterial activity value can be obtained when the Water hardness of the dilution water is smaller than the Water hardness of the papermaking water. From the above, it is shown that a flexible antibacterial paper having antibacterial performance can be stably manufactured when satisfying both: 1) A papermaking water having a Water hardness of 5° dH or more; and 2) The Water hardness of the dilution water is equal to or less than that of the papermaking water. Regarding to the item 2), it is more preferable that the Water hardness of the dilution water be less than that of the papermaking water.

Industrial Applicability

The antibacterial paper of the present invention has antibacterial performance and flexibility. According to the method for manufacturing method an antibacterial paper of the present invention, the high-quality antibacterial paper can be efficiently manufactured.

## Claims

1. An antibacterial paper containing an antibacterial agent,
wherein a calcium content per 0.1 g of the antibacterial paper is 500 ppm or more and 800 ppm or less.

2. The antibacterial paper according to claim 1, wherein the calcium content per 0.1 g of the antibacterial paper is 515 ppm or more and 700 ppm or less.

3. The antibacterial paper according to claim 1 or 2, wherein the antibacterial agent is a cationic antibacterial agent.

4. The antibacterial paper according to any one of claims 1 to 3, wherein the antibacterial agent is a benzalkonium salt represented by the following formula (1):

[Chem. 1]

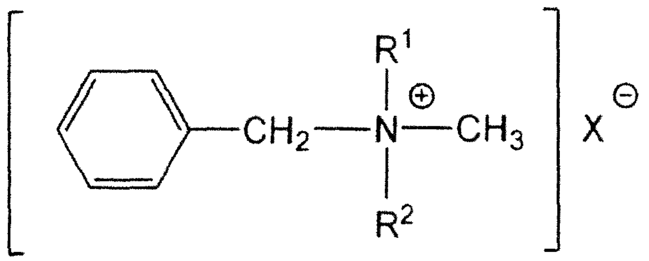

(1)

wherein $R^1$ and $R^2$ are the same or different and each represent a methyl group, an ethyl group, a straight-chain or branched-chain alkyl group having 8 or more and 20 or less carbon atoms, or a straight-chain or branched-chain alkenyl group having 8 or more and 20 or less carbon atoms; and $X^-$ represents a monovalent anion.

5. The antibacterial paper according to any one of claims 1 to 4, having an antibacterial activity value of 1 or more, the

antibacterial activity value being measured according to the bacteria solution absorption method in JIS L 1902: 2015 "Determination of antibacterial activity and efficacy of textile products".

**6.** The antibacterial paper according to claim 5. wherein the antibacterial activity value is 6 or less.

**7.** An absorbent product comprising the antibacterial paper according to any one of claims 1 to 6.

**8.** A method for manufacturing an antibacterial paper comprising:

a papermaking step of forming a wet paper web by wet papermaking using a papermaking water having a water hardness of 5° dH or more,
an antibacterial agent applying step of applying an antibacterial agent-containing liquid. which is prepared from an antibacterial agent and a dilution water thereof, to the wet paper web, and
a drying step of heating and drying the wet paper web after undergoing the antibacterial agent applying step. wherein the water hardness of the dilution water is equal to or less than the water hardness of the papermaking water.

**9.** The method for manufacturing an antibacterial paper according to claim 8, wherein the water hardness of the papermaking water is 6° dH or more and 8° dH or less.

**10.** The method for manufacturing an antibacterial paper according to claim 8 or 9, wherein the water hardness of the dilution water is less than the water hardness of the papermaking water.

**11.** The method for manufacturing an antibacterial paper according to any one of claims 8 to 10, wherein the water hardness of the dilution water is 4° dH or less.

**12.** The method for manufacturing an antibacterial paper according to any one of claims 8 to 11, wherein the water hardness of the dilution water/the water hardness of the papermaking water is 0.7 or less.

**13.** The method for manufacturing an antibacterial paper according to any one of claims 8 to 12, wherein the antibacterial agent is a cationic antibacterial agent.

## Patentansprüche

**1.** Ein antibakterielles Papier, das ein antibakterielles Mittel enthält,
wobei ein Calciumgehalt pro 0,1 g des antibakteriellen Papiers 500 ppm oder mehr und 800 ppm oder weniger beträgt.

**2.** Das antibakterielle Papier nach Anspruch 1, wobei der Calciumgehalt pro 0,1 g des antibakteriellen Papiers 515 ppm oder mehr und 700 ppm oder weniger beträgt.

**3.** Das antibakterielle Papier nach Anspruch 1 oder 2, wobei das antibakterielle Mittel ein kationisches antibakterielles Mittel ist.

**4.** Das antibakterielle Papier nach einem der Ansprüche 1 bis 3, wobei das antibakterielle Mittel ein Benzalkoniumsalz, dargestellt durch die folgende Formel (1), ist:

[Chem. 1]

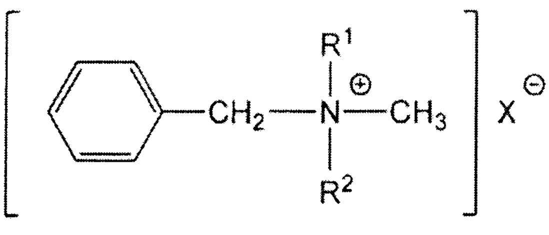

(1)

wobei $R^1$ und $R^2$ gleich oder verschieden sind und jeweils eine Methylgruppe, eine Ethylgruppe, eine geradkettige

oder verzweigte Alkylgruppe mit 8 oder mehr und 20 oder weniger Kohlenstoffatomen oder eine geradkettige oder verzweigte Alkenylgruppe mit 8 oder mehr und 20 oder weniger Kohlenstoffatomen darstellen; und $X^-$ ein einwertiges Anion darstellt.

**5.** Das antibakterielle Papier nach einem der Ansprüche 1 bis 4, welches einen antibakteriellen Aktivitätswert von 1 oder mehr aufweist, wobei der antibakterielle Aktivitätswert gemäß dem Bakterienlösungsabsorptionsverfahren in JIS L 1902: 2015 "Bestimmung antibakterieller Aktivität und Wirksamkeit von Textilwaren" gemessen wird.

**6.** Das antibakterielle Papier nach Anspruch 5, wobei der antibakterielle Aktivitätswert 6 oder weniger beträgt.

**7.** Ein absorbierendes Produkt, umfassend das antibakterielle Papier nach einem der Ansprüche 1 bis 6.

**8.** Ein Verfahren zur Herstellung eines antibakteriellen Papiers, umfassend:

einen Papierherstellungsschritt des Bildens einer nassen Papierbahn durch Nasspapierherstellung unter Verwendung eines Papierherstellungswassers mit einer Wasserhärte von 5° dH oder mehr,
einen Antibakterielles-Mittel-Aufbringungsschritt des Aufbringens einer antibakterielles Mittel enthaltenden Flüssigkeit, die aus einem antibakteriellen Mittel und einem Verdünnungswasser daraus hergestellt wird, auf die nasse Papierbahn und
einen Trocknungsschritt des Erwärmens und Trocknens der nassen Papierbahn, nachdem sie dem Schritt des Aufbringens eines antibakteriellen Mittels unterzogen wurde,
wobei die Wasserhärte des Verdünnungswassers gleich oder niedriger ist als die Wasserhärte des Papierherstellungswassers.

**9.** Das Verfahren zur Herstellung eines antibakteriellen Papiers nach Anspruch 8, wobei die Wasserhärte des Papierherstellungswassers 6° dH oder mehr und 8° dH oder weniger beträgt.

**10.** Das Verfahren zur Herstellung eines antibakteriellen Papiers nach Anspruch 8 oder 9, wobei die Wasserhärte des Verdünnungswassers niedriger ist als die Wasserhärte des Papierherstellungswassers.

**11.** Das Verfahren zur Herstellung eines antibakteriellen Papiers nach einem der Ansprüche 8 bis 10, wobei die Wasserhärte des Verdünnungswassers 4° dH oder weniger beträgt.

**12.** Das Verfahren zur Herstellung eines antibakteriellen Papiers nach einem der Ansprüche 8 bis 11, wobei die Wasserhärte des Verdünnungswassers/die Wasserhärte des Papierherstellungswassers 0,7 oder weniger beträgt.

**13.** Das Verfahren zur Herstellung eines antibakteriellen Papiers nach einem der Ansprüche 8 bis 12, wobei das antibakterielle Mittel ein kationisches antibakterielles Mittel ist.

## Revendications

**1.** Papier antibactérien contenant un agent antibactérien, dans lequel la teneur en calcium pour 0,1 g du papier antibactérien est de 500 ppm ou plus et 800 ppm ou moins.

**2.** Papier antibactérien selon la revendication 1, dans lequel la teneur en calcium pour 0,1 g du papier antibactérien est de 515 ppm ou plus et 700 ppm ou moins.

**3.** Papier antibactérien selon la revendication 1 ou 2, dans lequel l'agent antibactérien est un agent antibactérien cationique.

**4.** Papier antibactérien selon l'une quelconque des revendications 1 à 3, dans lequel l'agent antibactérien est un sel de benzalkonium représenté par la formule (1) suivante :

[formule chimique 1]

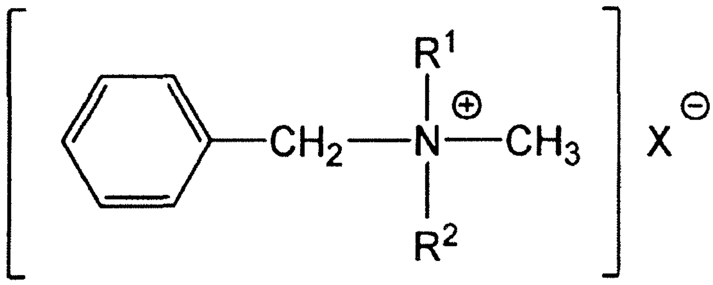

(1)

dans laquelle $R^1$ et $R^2$ sont identiques ou différents et représentent chacun un groupe méthyle, un groupe éthyle, un groupe alkyle à chaîne droite ou à chaîne ramifiée ayant 8 ou plus et 20 ou moins atomes de carbone, ou un groupe alcényle à chaîne droite ou à chaîne ramifiée ayant 8 ou plus et 20 ou moins atomes de carbone ; et $X^-$ représente un anion monovalent.

**5.** Papier antibactérien selon l'une quelconque des revendications 1 à 4, ayant une valeur d'activité antibactérienne de 1 ou plus, la valeur d'activité bactérienne étant mesurée conformément à la méthode d'absorption de solution de bactéries dans la norme JIS L 1902 : 2015 "Détermination d'activité antibactérienne et efficacité de produits textiles".

**6.** Papier antibactérien selon la revendication 5, dans lequel la valeur d'activité antibactérienne est de 6 ou moins.

**7.** Produit absorbant comprenant le papier antibactérien selon l'une quelconque des revendications 1 à 6.

**8.** Méthode pour fabriquer un papier antibactérien, comprenant :

une étape de fabrication de papier en formant une bande de papier humide par fabrication de papier humide utilisant une eau de fabrication de papier ayant une dureté de l'eau de 5° dH ou plus,
une étape d'application d'agent antibactérien en appliquant un liquide contenant un agent antibactérien, qui est préparé à partir d'un agent antibactérien et d'une eau de dilution de celui-ci, à la bande de papier humide, et
une étape de séchage en chauffant et séchant la bande de papier humide après qu'elle a été soumise à l'étape d'application d'agent antibactérien,
dans laquelle la dureté de l'eau de dilution est égale ou inférieure à la dureté de l'eau de fabrication de papier.

**9.** Méthode pour fabriquer un papier antibactérien selon la revendication 8, dans laquelle la dureté de l'eau de fabrication de papier est de 6° dH ou plus et 8° dH ou moins.

**10.** Méthode pour fabriquer un papier antibactérien selon la revendication 8 ou 9, dans laquelle la dureté de l'eau de dilution est inférieure à la dureté de l'eau de fabrication de papier.

**11.** Méthode pour fabriquer un papier antibactérien selon l'une quelconque des revendications 8 à 10, dans laquelle la dureté de l'eau de dilution est de 4° dH ou moins.

**12.** Méthode pour fabriquer un papier antibactérien selon l'une quelconque des revendications 8 à 11, dans laquelle le rapport dureté de l'eau de dilution / dureté de l'eau de fabrication de papier est de 0,7 ou moins.

**13.** Méthode pour fabriquer un papier antibactérien selon l'une quelconque des revendications 8 à 12, dans laquelle l'agent antibactérien est un agent antibactérien cationique.

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- JP 2016168133 A


### Non-patent literature cited in the description


- **W.S. MUELLER ; O. B. SEELY.** *Soap & Sanitary Chemicals,* 1951, vol. 27 (6), 131